Europäisches Patentamt

European Patent Office

Office européen des brevets

(11) Veröffentlichungsnummer: **0 208 201**
**A2**

(12) **EUROPÄISCHE PATENTANMELDUNG**

(21) Anmeldenummer: 86108647.8

(22) Anmeldetag: 25.06.86

(51) Int. Cl.⁴: **A 61 B 5/10**

(30) Priorität: 08.07.85 DE 3524354

(43) Veröffentlichungstag der Anmeldung:
14.01.87 Patentblatt 87/3

(84) Benannte Vertragsstaaten:
AT BE CH DE FR GB IT LI NL SE

(71) Anmelder: Wienert, Volker, Prof.Dr.med.
Elsenborn 53
D-5100 Aachen(DE)

(71) Anmelder: Sick, Hinrich, Prof. Dr.
Sielbecker Landstrasse 48
D-2420 Eutin(DE)

(72) Erfinder: Wienert, Volker, Prof.Dr.med.
Elsenborn 53
D-5100 Aachen(DE)

(72) Erfinder: Sick, Hinrich, Prof. Dr.
Sielbecker Landstrasse 48
D-2420 Eutin(DE)

(74) Vertreter: Biermann, Wilhelm, Dr.-Ing.
Morillenhang 39
D-5100 Aachen(DE)

(54) Vorrichtung zum Erstellen eines Plethysmogramms.

(57) Gegenstand der Erfindung ist ein eine Meßeinrichtung zur Erfassung der Volumenänderung eines Körperteils und ein Auswertegerät umfassender Plethysmograph. Die Meßeinrichtung besteht aus einem lichtoptischen Empfänger, der den in einem festgelegten Abstand (A) von dem lichtoptischen Empfänger angeordneten Körperteil flächenmäßig erfaßt. Das Auswertegerät besteht aus einer Einrichtung, die die Größe der lichtoptisch erfaßten Fläche quantitativ auswertet.

Gemäß einem Ausführungsbeispiel ist der lichtoptische Empfänger (2) eine Videokamera. Das Auswertegrät umfaßt einen Mikrocomputer (9), der die Größe der Fläche (6) des gleichzeitig auf einem Monitor (5) abgebildeten Körperteils (1) numerisch auswertet und das Plethysmogramm auf einem Anzeigegerät (11) ausdruckt.

Fig. 1

0208201

Die Erfindung betrifft eine Vorrichtung zum Erstellen eines Plethysmogramms, mit einer Meßeinrichtung zur Erfassung der Volumenänderung eines Körperteils und einem die Meßgröße auswertenden und als Funktion der Zeit darstellenden Auswerte- und Anzeigegerät.

Bei der Plethysmographie werden die Volumenänderungen eines Körperteils, beispielsweise die Volumenänderungen, die auf Änderungen der Blutfülle dieses Körperteils zurückgehen, quantitativ erfaßt. Grundsätzlich läßt sich die Plethysmographie auf alle Körperteile oder Abschnitte von Körperteilen, oder auch auf den Körper in seiner Gesamtheit anwenden. Besondere Bedeutung hat sie unter anderem als Diagnostizierverfahren bei Verdacht auf einen Venenverschluß im Bereich des Oberschenkels oder des Unterschenkels erlangt. Zur Aufnahme des Plethysmogramms wird in diesem Fall mit Hilfe einer am Oberschenkel angebrachten Staumanschette der Blutrückfluß durch die Venen unterbunden, während die arterielle Blutzufuhr nicht behindert wird. Durch den einsetzenden Blutstau erfolgt eine Volumenvergrößerung des Beins unterhalb der Staumanschette. Die bei Aufhebung des Staudrucks erfolgende zeitliche Volumenabnahme läßt Rückschlüsse auf den Zustand der Venen zu.

Bei einer bekannten Vorrichtung zur Aufnahme eines Plethysmogramms besteht die Meßeinrichtung für die Erfassung der Volumenänderung aus einem Dehnungsmeßstreifen, beispielsweise einem mit Quecksilber gefüllten Schlauch, der um den zu untersuchenden Körperteil herumgelegt wird und der in Abhängigkeit von dem sich ändernden Umfang des Körperteils seinen Widerstand ändert. Bei einer anderen bekannten Vorrichtung wird der zu untersuchende Körperteil in ein mit Wasser gefülltes Gefäß eingetaucht, und die durch die Volumenänderung des Körperteils erfolgende Veränderung des Wasserniveaus wird als Maß für die Volumenvergrößerung ausgewertet.

Bei den bekannten Methoden erfolgt die Messung der Volumen-

0208201

änderung des Körperteils durch direkten Kontakt eines Meßfühlers bzw. eines druckübertragenden Mediums mit dem Körperteil. Dadurch wird zwangsläufig auf den zu untersuchenden Körperteil ein Gegendruck ausgeübt, der sowohl die Blutzufuhr selbst behindert als auch die ungehinderte Volumenvergrößerung des betreffenden Körperteils, und damit auch die erhaltenen Meßergebnisse, beeinträchtigt.

Der Erfindung liegt die Aufgabe zugrunde, eine Vorrichtung der eingangs genannten Art zu schaffen, die eine Messung der Volumenänderung eines Körpers oder eines Körperteils ohne jeden Kontakt des Körpers mit einem Meßfühler oder mit einem Übertragungsmedium gestattet, und die auf diese Weise jede durch die Meßmethode bedingte Beeinträchtigung der Blutzufuhr oder der Volumenänderung sicher vermeidet.

Gemäß der Erfindung wird diese Aufgabe dadurch gelöst, daß die Meßeinrichtung zur Erfassung der Volumenänderung des Körperteils aus einem lichtoptischen Empfänger besteht, der den in einem festgelegten Abstand von dem lichtoptischen Empfänger angeordneten Körper oder Körperteil, bzw. einen definierten Abschnitt dieses Körperteils, flächenmäßig erfaßt, und daß das Auswertegerät aus einer Einrichtung besteht, die die Größe der lichtoptisch erfaßten Fläche des Körperteils auswertet.

Ein wesentlicher Grundgedanke der Erfindung besteht also darin, die Messung der Volumenänderung des Körperteils berührungsfrei auf lichtoptischem Wege vorzunehmen. Dabei hat es sich gezeigt, daß das zweidimensionale Bild oder Schattenbild des betreffenden Körperteils, das heißt die durch die Projektion des Körperteils in einer Richtung hervorgerufene flächenmäßige Abbildung des Körperteils, für die Bestimmung der Volumenänderung ausreicht. Zweckmäßigerweise erfolgt dabei die optische Abbildung des betreffenden Körperteils von einer Richtung her, die etwa senkrecht zur Richtung der größten Volumenänderung des Körperteils verläuft. Bei der Venenverschluß-Plethysmographie z.B. läßt sich die optische Abbildung der Wade für das

0208201

Plethysmogramm besonders gut auswerten, wenn das Bein horizontal mit der Wade nach unten gelagert wird und die optische Beobachtung von der Seite her erfolgt.

Grundsätzlich ist es für die Auswertung der lichtoptischen Abbildung wichtig, daß ein kontrastreiches Bild des Körpers bzw. des betreffenden Körperteils und des Hintergrundes erzeugt wird. Dabei kann der Körperteil vor einer großflächigen Beleuchtungseinrichtung oder vor einem anderen hellen Hintergrund angeordnet sein, so daß von dem lichtoptischen Empfänger ein dunkles Bild des Körperteils vor einem hellen Hintergrund erfaßt wird. Stattdessen kann der zu untersuchende Körperteil auch zwischen einer nahezu punktförmigen Lichtquelle und einem transparenten Schirm angeordnet sein, so daß von dem lichtoptischen Empfänger das Schattenbild des Körperteils auf dem im übrigen von der Lichtquelle erleuchteten transparenten Schirm erfaßt wird. Es ist jedoch ebenso möglich, den Körperteil auf der von dem lichtoptischen Empfänger erfaßten Seite zu beleuchten, und durch den lichtoptischen Empfänger ein kontrastreiches Bild des hell erleuchteten Körperteils vor einem dunklen Hintergrund zu erfassen.

Die Auswertung der Bildfläche kann ebenfalls auf verschiedene Weise erfolgen. Gemäß einer ersten Ausführungsform kann beispielsweise mit Hilfe einer Sammeloptik die gesamte von einer definierten, die Abbildung des entsprechenden Körperteils aufweisenden Fläche ausgehende Lichtmenge erfaßt und ihre Änderung gemessen werden. In diesem Fall müssen entweder die Größe des Meßfeldes und die Helligkeit des Hintergrundes und des Körperteils konstant bleiben, oder es muß durch Anwendung von moduliertem Licht der Einfluß der Umgebungs- bzw. der Tageslichtbeleuchtung ausgeschaltet werden, wobei in diesem Fall die Lichtmenge bzw. Lichtintensität jeweils über eine gleiche Anzahl von Modulationsperioden erfaßt wird. Die erfaßte Gesamtlichtmenge wird einem lichtelektrischen Wandler zugeführt, der ein der Flächenänderung des abgebildeten Körperteils proportionales elektrisches Signal liefert. Bei dieser Methode kann die Auswertung des Bildes entweder mit Hilfe eines analogen Auswerteverfahrens oder auch digital, beispielsweise unter Verwendung einer geeigneten Interfaceelektronik mittels eines Mikrocomputers erfolgen.

Gemäß einer anderen Ausführungsform wird die Bildfläche mit Hilfe einer Videokamera erfaßt. Die Auswertung des Bildes erfolgt mit Hilfe eines digitalen Bildspeichers und eines Mikrocomputers, der die Größe der Fläche des abgebildeten Körperteils numerisch auswertet.

Ein Ausführungsbeispiel der Erfindung, das nach diesem zuletzt genannten Verfahren arbeitet, wird nachfolgend anhand der Zeichnungen näher beschrieben.

Von den Zeichnungen zeigt

Fig. 1    den Aufbau eines erfindungsgemäßen Plethysmographen in einer schematischen Darstellung,

Fig. 2    den mechanisch-optischen Teil des Plethysmographen in Form einer perspektivischen Gesamtansicht, und

Fig. 3    ein mit der dargestellten Vorrichtung aufgenommenes Plethysmogramm.

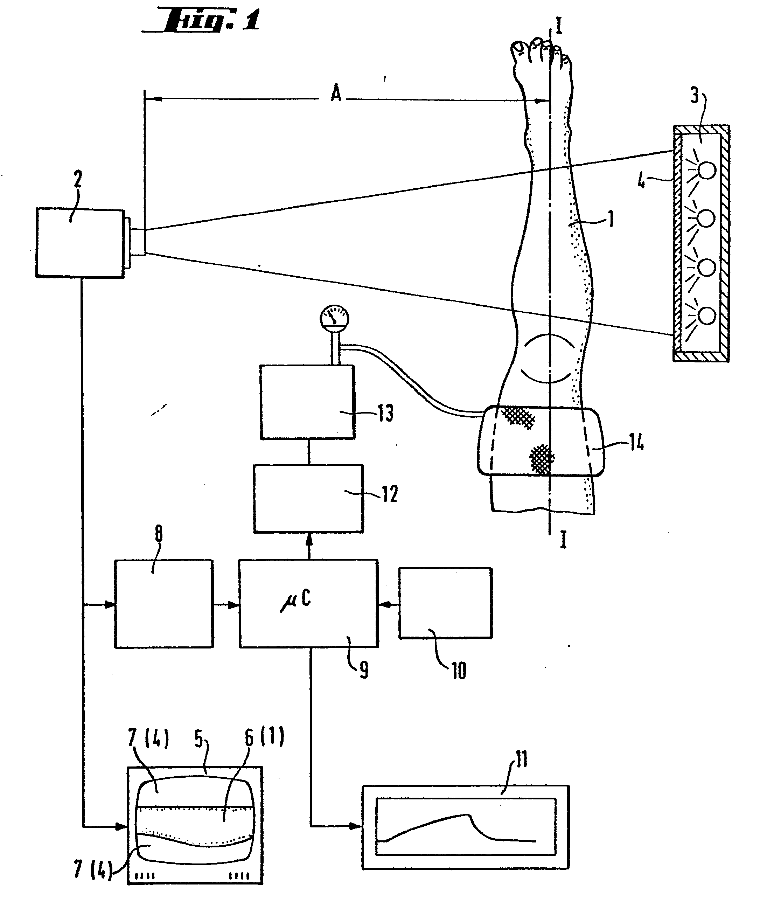

In Fig. 1 ist der Aufbau eines mit einer Videokamera arbeitenden, für die Venenverschluß-Plethysmographie geeigneten Plethysmographen mit seinen wesentlichen Komponenten schematisch dargestellt. Das zu untersuchende Bein 1 wird in einer definierten Ebene I-I, die sich in einem festgelegten Abstand A vor der Optik der Videokamera 2 befindet, derart horizontal gelagert, daß das Schienbein nach oben und die Wade senkrecht nach unten weisen. Von der Videokamera 2 aus gesehen befindet sich hinter dem Bein 1 die Beleuchtungseinrichtung 3, die aus einem vorn durch eine Milchglasscheibe 4 abgeschlossenen, mit Lampen versehenen Gehäuse besteht.

Es kann für diese Beleuchtungseinrichtung 3 ein Leuchtkasten verwendet werden, wie er für die Betrachtung von Röntgenfilmen üblich ist. Vor der Videokamera 2 wird auf diese Weise der Abschnitt des Beines von etwa unterhalb der Kniescheibe bis etwa 10 cm oberhalb des Knöchels vor einem hellen Hintergrund aufgenommen, und es ergibt sich so ein kontrastreiches Bild, bei dem optisch der senkrechte Längsschnitt des Beines in seiner größten Ausdehnung als dunkle Fläche von einer hellen Fläche umgeben dargestellt ist. Die Videokamera 2 erfaßt also gewissermaßen das Schattenbild des in einer bestimmten Richtung ausgerichteten Beins.

Es hat sich als vorteilhaft erwiesen, bei Verwendung einer Videokamera die Anordnung so zu treffen, daß die Zeilenspur senkrecht zur Beinrichtung verläuft. Zu diesem Zweck wird die Videokamera 2 im Vergleich zu ihrer normalen Ausrichtung um $90^o$ um die optische Achse gedreht, da das Bein in der horizontalen Lage angeordnet werden muß.

Das von der Videokamera 2 aufgenommene Bild des Beins läßt sich auf dem Monitor 5 zu Kontrollzwecken visuell betrachten. Man erkennt auf dem Monitor 5 das dunkle Schattenbild 6 des Beins 1 zwischen den hellen Flächen 7 der hellen Milchglasscheibe 4. Die Fläche des auf dem Monitor 5 erkennbaren Bildes 6 des Beins wird in festgelegten Zeitintervallen in der Auswerteeinrichtung quantitativ ausgewertet und registriert.

Die Auswerteeinrichtung umfaßt eine Interface-Elektronik 8 und einen Mikrocomputer 9. Die Interface-Elektronik 8 dient dazu, das von der Videokamera 2 gelieferte Signal so aufzubereiten, daß es von dem Mikrocomputer 9 ausgewertet werden kann. Sie enthält unter anderem einen sehr schnellen AD-Wandler, einen Videoprozessor und einen Bildspeicher, in dem das zu den festgelegten Zeitpunkten von der Videokamera aufgenommene Bild für die Auswertung durch den Mikrocomputer 9 abgespeichert wird. Die Auswertung besteht im einfachsten Fall aus einer planimetrischen Vermessung der Fläche des abgebildeten Beins und der Bestimmung deren zeitabhängigen Änderung. Durch Anwendung empirisch ermittelter Umrechnungsfaktoren oder -algorithmen läßt sich anstelle einer reinen Flächenbestimmung auch eine echte Volumenmessung durchführen.

Ebenfalls an den Mikrocomputer 9 angeschlossen ist ferner ein Diskettenlaufwerk 10, mit dessen Hilfe im vorliegenden Fall ein geeignetes Programm für die Durchführung der Venenverschluß-Plethysmographie in den Mikrocomputer 9 eingegeben wird. Das Programm für die Durchführung der Venenverschluß-Plethysmographie kann so angelegt sein, daß in der Stauungsphase das Bild in größeren Zeitintervallen ausgewertet wird als in der Entstauungsphase, so daß auf dem Plethysmogramm die Zeitskala in der Entstauungsphase gedehnt ist.

Mit dem Mikrocomputer 9 ist der Drucker 11 verbunden, der das Plethysmogramm unmittelbar erstellt. Ferner kann von dem Mikrocomputer 9 wahlweise eine Steuereinheit 12 angesteuert werden, die einen Druckregler 13 für die Steuerung des pneumatischen Drucks in der Staumanschette 14 betätigt. Mit Hilfe des Mikrocomputers 9, der Steuereinheit 12 und des Druckreglers 13 kann beispielsweise dafür gesorgt werden, daß die Staumanschette 14 erst dann unter Druck gesetzt wird, wenn die gesamte Vorrichtung für die Durchführung der Messung vorbereitet ist. Ebenso kann hiermit die Staumanschette 14 automatisch entlüftet werden, sobald der Mikrocomputer 9 erkennt, daß das Beinvolumen nicht mehr zunimmt.

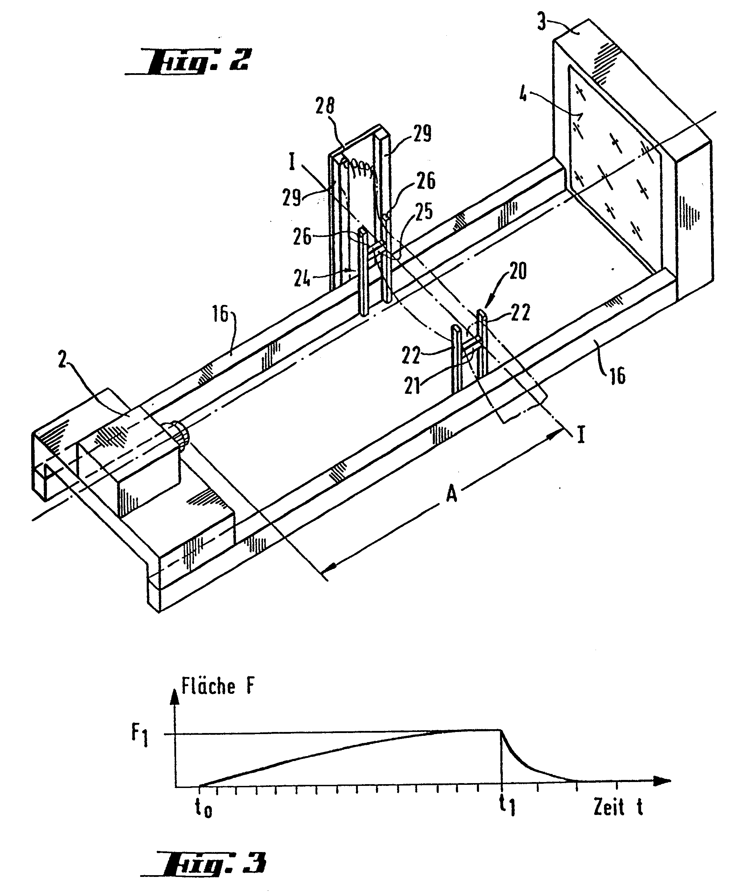

Die mechanischen Bestandteile des Plethysmographen, nämlich die Videokamera 2 und die Beleuchtungseinrichtung 3, sind in fester Zuordnung zueinander auf einem starren Rahmen 16 in Art einer optischen Bank mit geringer Präzision angeordnet. Auf dem Rahmen 16 ist ferner, wie aus Fig. 2 ersichtlich ist, an einer festgelegten Stelle im Abstand A von der Videokamera 2, die am anderen Ende der optischen Bank auf dem Rahmen 16 fest angeordnet ist, eine Positioniereinrichtung für die Lagerung und gegebenenfalls Fixierung des Beins 1 des Patienten angeordnet. Diese Positioniereinrichtung umfaßt eine Stütze 20 für die Positionierung des Beins im Kniebereich, wobei sie im wesentlichen aus einem die Höhe des Beines festlegenden horizontalen Stützkörper 21 und zwei das Bein seitlich begrenzenden Stützen 22 besteht. Im Bereich des Fußes wird das Bein durch eine Stütze 24 in vertikaler und in horizontaler Richtung fixiert, wobei diese Stütze 24 wiederum einen horizontalen Stützkörper 25 und zwei vertikale Stützen 26 umfaßt. Außerdem umfaßt die Positioniereinrichtung noch eine Platte 28, gegen die der Fuß zur Anlage kommt, und auf der der Fuß durch die beiden Begrenzungskörper 29 in vertikaler Ausrichtung fixiert wird. Vor der Positioniereintichtung 20, 24, 28 ist eine nicht dargestellte Liege angeordnet, auf der der Patient während der Aufnahme des Plethysmogramms liegt. Außerdem kann eine weitere Stützvorrichtung für das andere Bein vorgesehen sein, auf der dieses außerhalb des Strahlenganges des lichtoptischen Empfängers während der Untersuchung ruht.

Die in Fig. 2 dargestellte Vorrichtung kann gegebenenfalls durch eine nicht dargestellte Anordnung ergänzt werden, bei der die Videokamera 2 auf einem verschwenkbaren Ständer angeordnet ist, der die Verschwenkung des Rahmens 16 mit der Videokamera und der Beleuchtungseinrichtung um die Längsachse des zu untersuchenden Beins gestattet. Dabei reicht eine Verschwenkungsmöglichkeit von $90^{o}$ aus, um das Schattenbild des Beins in zwei senkrecht aufeinanderstehenden Richtungen und in den entsprechenden Zwischenstellungen zu erfassen. Auf diese Weise läßt sich bei entsprechender Programmierung des Mikrocomputers eine vollständige Volumenmessung des Beins durchführen. Gegebenenfalls kann ein solcher verschwenkbar angeordneter lichtoptischer Empfänger zwecks Raumersparnis durch beweglich angeordnete Spiegel ergänzt werden, die das aus verschiedenen Beobachtungsrichtungen sichtbare Bild des Beins auf den lichtoptischen Empfänger, d.h. im vorliegenden

0208201

*Fall auf die Videokamera, richten.*

*Ein mit der beschriebenen Einrichtung aufgenommenes und von dem Drucker 11 geschriebenes Plethysmogramm ist in Fig. 3 wiedergegeben. In Abhängigkeit von der Zeit T ist hier die beobachtete Fläche F aufgetragen. Im Zeitpunkt $t_0$, in dem die Staumanschette den notwendigen Staudruck aufbringt, um den Rückfluß des Blutes zu unterbinden, beginnt die beobachtete Abbildungsfläche F sich zu vergrößern und erreicht nach einer gewissen Zeit die Größe $F_1$. In diesem Zustand ist der Blutstau so groß, daß keine weitere Blutzufuhr erfolgt, so daß die Fläche F sich nicht mehr verändert. Wenn der Mikrocomputer feststellt, daß die Fläche $F_1$ sich während eines gegebenen Zeitraums nicht mehr verändert, wird über die Steuereinheit 12 und über den Druckregler 13 (Fig. 2) die Staumanschette zum Zeitpunkt $t_1$ schlagartig entlüftet. Das gestaute Blut fließt daraufhin je nach dem Zustand der Venen mehr oder weniger schnell ab, so daß die Kurve der Flächengröße nach dem Zeitpunkt $t_1$ mehr oder weniger rasch abfällt. Zur besseren Auswertung ist nach dem Zeitpunkt $t_1$ die Zeitskale gedehnt. Der Verlauf der Kurve nach dem Zeitpunkt $t_1$ läßt Rückschlüsse auf den Zustand der Venen zu.*

1

Vorrichtung
zum Erstellen eines Plethysmogramms

Patentansprüche

1. Vorrichtung zum Erstellen eines Plethysmogramms, mit einer
Meßeinrichtung zur Erfassung der Volumenänderung eines
Körperteils und einem die Meßgröße auswertenden und als
Funktion der Zeit darstellenden Auswerte- und Anzeigegerät,
d a d u r c h   g e k e n n z e i c h n e t , daß die Meßeinrichtung zur Erfassung der Volumenänderung des Körperteils aus einem lichtoptischen Empfänger (2) besteht, der
den in einem festgelegten Abstand (A) von dem lichtoptischen
Empfänger (2) angeordneten Körperteil (1), bzw. einen definierten Abschnitt dieses Körperteils (1), flächenmäßig
erfaßt, und daß das Auswertegerät aus einer Einrichtung besteht, die die Größe der lichtoptisch erfaßten Fläche auswertet.

2. Vorrichtung nach Anspruch 1, dadurch gekennzeichnet, daß
der Körperteil (1) vor einer großflächigen Beleuchtungseinrichtung (3) angeordnet ist, und daß von dem lichtoptischen Empfänger ein kontrastreiches Bild des zu untersuchenden unbeleuchteten Körperteils vor einem hellen Hintergrund erfaßt wird.

3. Vorrichtung nach Anspruch 1, dadurch gekennzeichnet, daß
der Körperteil zwischen einer nahezu punktförmigen Lichtquelle und einem transparenten Schirm angeordnet ist, und
daß von dem auf den transparenten Schirm ausgerichteten lichtoptischen Empfänger das Schattenbild des Körperteils
erfaßt wird.

4. Vorrichtung nach Anspruch 1, dadurch gekennzeichnet, daß
der Körperteil auf der von dem lichtoptischen Empfänger
erfaßten Seite beleuchtet wird, und daß von dem lichtoptischen Empfänger ein kontrastreiches Bild des hell erleuchteten zu untersuchenden Körperteils vor einem dunklen
Hintergrund erfaßt wird.

5. Vorrichtung nach einem der Ansprüche 2 bis 4, dadurch gekennzeichnet, daß der lichtoptische Empfänger eine auf die
zu beobachtende Fläche von konstanter Größe ausgerichtete
Sammeloptik und einen die von der Sammeloptik erfaßte Gesamtlichtmenge messenden lichtelektrischen Wandler umfaßt,
der ein der Flächengröße des abgebildeten Körperteils proportionales elektrisches Signal liefert.

6. Vorrichtung nach einem der Ansprüche 2 bis 4, dadurch gekennzeichnet, daß der lichtoptische Empfänger aus einer die
zu beobachtende Fläche von konstanter Größe erfassenden
Videokamera (2) besteht, und daß das Auswertegerät einen
Mikrocomputer (9) umfaßt, der die Größe der Fläche (6) des
abgebildeten Körperteils (1) numerisch auswertet.

7. Vorrichtung nach einem oder mehreren der Ansprüche 1 bis 6,
dadurch gekennzeichnet, daß die den lichtoptischen Empfänger
bildende Videokamera (2), die Beleuchtungseinrichtung (3)
und eine Fixiervorrichtung für den zu untersuchenden Körperteil (1) auf einem gemeinsamen Rahmen (16) angeordnet sind.

8. Vorrichtung nach Anspruch 7, dadurch gekennzeichnet, daß
die Fixiervorrichtung für ein Bein (1) aus einer das Bein
(1) im Bereich des Knies in horizontaler und in vertikaler
Richtung festlegenden Stütze (20), einer den Fuß in horizontaler und in vertikaler Richtung festlegenden Stütze (24),

0208201

sowie einer den Fuß in vertikaler Ausrichtung haltenden Haltevorrichtung (28) besteht.

9. Vorrichtung nach einem oder mehreren der Ansprüche 6 bis 8, dadurch gekennzeichnet, daß mit dem Mikrocomputer (9) ein Drucker (11) verbunden ist, der während oder nach der Untersuchung unmittelbar das die zeitliche Veränderung der beobachteten Fläche darstellende Plethysmogramm schreibt.

10. Vorrichtung nach einem oder mehreren der Ansprüche 6 bis 9, gekennzeichnet durch einen Monitor (5) zur optischen Wiedergabe des von der Videokamera (2) aufgenommenen Bildes (6).

11. Vorrichtung nach einem oder mehreren der Ansprüche 6 bis 10, gekennzeichnet durch eine von dem Mikrocomputer (9) angesteuerte Steuereinheit (12) für die automatische Steuerung eines die Staumanschette (14) betätigenden Druckreglers (13).

12. Vorrichtung nach einem oder mehreren der Ansprüche 1 bis 11, dadurch gekennzeichnet, daß der lichtoptische Empfänger (2) auf einem verschwenkbaren Ständer angeordnet ist, der eine Verschwenkung des lichtoptischen Empfängers (2) in einem gleichbleibenden Abstand um den zu untersuchenden Körperteil herum erlaubt.

**Fig. 1**

_Fig. 2_

_Fig. 3_